# EUROPEAN PATENT SPECIFICATION

(11) **EP 4 316 550 B1**
(45) Date of publication and mention of the grant of the patent: **25.03.2026**
(21) Application number: 22306185.4
(22) Date of filing: 04.08.2022
(51) Int. Cl.: A61M 5/31, A61M 39/20

(54) **TIP CAP ASSEMBLY FOR AN INJECTION SYSTEM**
SPITZENKAPPENANORDNUNG FÜR EIN INJEKTIONSSYSTEM
ENSEMBLE BOUCHON D'EXTRÉMITÉ UN SYSTÈME D'INJECTION

(43) Date of publication of application: 07.02.2024
(73) Proprietor: Becton Dickinson France, 38800 Le Pont de Claix (FR)
(72) Inventor: RIVIER, Cédric, 38340 Voreppe (FR); NICOLAS, Maxime, 38170 Seyssinet-Pariset (FR); VAXELAIRE, Jeremie, 38000 Grenoble (FR); OZTURK, Senturk, 38130 Echirolles (FR); CIBOULET, Antoine, 38000 Grenoble (FR)
(74) Representative: Regimbeau

(56) References cited:
- EP-A1- 2 862 587
- EP-A1- 3 269 418
- EP-A1- 3 868 437
- US-A1- 2019 046 736
- US-A1- 2019 351 212

## Description

### TECHNICAL FIELD

The present disclosure relates to a tip cap assembly for closing a fluid passageway of a medical injection device and to the injection system including the injection device and the tip cap assembly.

### BACKGROUND

Current medicine uses a wide range of injection devices to deliver fluids into the body of patients. For example, such injection systems may include auto-injectors, medical pens or syringes. Conventional syringes are used because of their common availability, ease of use and limited cost. Syringes may be prefilled or prefillable. Syringes comprise a longitudinal barrel with an open proximal end and a substantially closed distal end including a distally projecting tip. The fluid intended to be injected can be stored in the syringe barrel, and in this case, the open proximal end is closed by a stopper in sliding fluid-tight engagement within the barrel and actuated by a plunger rod. The tip is provided with a fluid passageway extending therethrough to allow the injection of the fluid when a distal pressure is applied on the plunger. The tip can be provided with an attached needle or can be of a luer type, meaning needle-free. Syringe barrels may be made of glass or plastic. Glass may be chosen for its chemical neutrality and low gas permeability whereas plastic may be chosen for its resistance to shocks.

Almost all fluids can be injected with a syringe. For example, a fluid can be a pharmaceutical solution such as a drug, a vaccine, vitamins or dietary minerals. Syringes are also useful to inject diagnostic solutions, cosmetic fluids, including gels such as hyaluronic acid or silicone compositions. The injection can be performed in every part of the body including skin, hypodermis, muscle and veins, depending on the application.

Needle-free syringes may be provided with an adaptor to which a device for allowing the transfer of the fluid within the syringe to be dispensed therefrom. In the case of a plastic syringe, the adaptor may be integrally formed with the syringe body. For glass syringes, the adaptor may be separately formed and coupled to the syringe body.

During the time between the filling of the syringe and its use, needle-free syringes are provided with a tip cap to close a distally extending tip. Examples of tip caps are provided in WO2018/011259 and US11013865. US 2019/046736 teaches a tip cap according to the preamble of claim 1. The tip cap is engaged with the adaptor provided on the syringe body. As fluids are stored in the prefilled syringe for an extended period of time, such as 6 to 18 months before injection, the injection system remains sealed during this period. The quality of the sealing between the tip cap and the syringe is important as a deficient sealing could damage the nature or the purity of the fluids, leading to waste of valuable fluids, potential unacceptable risks for the patients and potential unacceptable risks for the medical staff according to the nature of the pharmaceutical compositions stored inside the syringes.

For instance, the outside surface of the distally extending tip should be preserved from contaminants, such as dust or micro-organisms, which may migrate from the tip to the fluid passageway. If these contaminants are injected with the medicinal fluid to a patient, it may trigger an inappropriate immune response, lessen the treatment efficacy and decrease the patient's trust into his treatment. Thus, displacement, in whole or in part, of the tip cap intended to close the fluid passageway may result in contamination of the medicinal fluid.

The force applied in an attempt to engage a rigid tip cap with the adaptor may be directly transmitted to the adaptor. Crack(s) may be formed in the adaptor and/or the tip cap made of a rigid material due to excessive mechanical stress generated during or after the assembly. Such crack(s) may lead to a poor connection of the adaptor with an intravenous (IV) line, needle hub, or other device for later assembly, may lead to contamination of the fluid within the syringe, and/or may lead to unintended exposure of a user to the fluid within the syringe.

Moreover, the syringe should be opened easily when required and the tip cap should be removed without excessive effort. A sticking phenomenon may occur when a tip cap is plugged on the tip of a syringe. It has been observed that when two materials are compressed together over an extended period of time, such phenomenon of sticking could occur and prevent a fast and easy opening of a prefilled syringe. Consequently, a tip cap difficult to open would lead to the rejection of the prefilled syringes before use and would constitute an unacceptable economic loss. This could also lead to the death or severe injury of patients requiring an immediate injection.

### BRIEF SUMMARY

An objective of the present disclosure is to a tip cap assembly for closing a fluid passageway extending through a tip of a container of a medical injection device overcoming one or more of the foregoing drawbacks.

The tip cap assembly comprises a rigid outer cap and an elastomeric inner cap. The rigid out cap has a distal portion and a proximal portion. An inner surface of the distal portion defines a cavity configured to retain the elastomeric inner cap therein. An inner surface of the proximal portion defines a cavity configured to surround the tip of the container when the tip cap assembly is assembled with the container. An elastomeric sealing ring is bonded to the inner surface of the proximal portion of the rigid outer cap. An inner surface of the sealing ring configured to engage the tip when the tip cap assembly is assembled with the container, the inner surface of the sealing ring comprises a plurality of ribs, and a surface of the ribs is configured to engage an outer surface of the tip of the container. The elastomeric inner cap is disposed within the cavity of the distal portion of the rigid outer cap.

Certain preferred but non-limiting features of the tip cap assembly described above are the following, taken individually or in combination:
an inner diameter of the sealing ring is less than an outer diameter of the tip on which the tip cap assembly is configured to be disposed;
a height of the sealing ring is less than a height of the tip on which the tip cap assembly is configured to be disposed, preferably the height of the sealing ring is 40% of the height of the tip;
the elastomeric sealing ring comprises a thermoplastic elastomer; and/or
the rigid outer cap is made of a rigid polymer, the rigid polymer preferably being polypropylene, polyethylene, polyvinylchloride, polystyrene, polycarbonate, acrylonitrile butadiene styrene or styrene acrylonitrile.

A method of forming the tip cap assembly includes comolding the sealing ring to the inner surface of the proximal portion of the rigid outer cap.

Another objective of the present disclosure is to an injection device comprising the tip cap assembly as previously described, a container, and an adaptor. The container has a barrel and a tip distally projecting from the barrel. The tip comprises a fluid passageway extending longitudinally therethrough. The adaptor is disposed circumferentially about the tip of the container.

Certain preferred but non-limiting features of the injection device described above are the following, taken individually or in combination:
an outer surface of the proximal portion of the rigid outer cap has a threaded surface;
the adaptor comprises a distal portion having an inner surface that is threaded, the threaded surface of the proximal portion of the rigid outer cap engaged with the threaded inner surface of the adaptor;
the elastomeric sealing ring of the tip cap assembly is radially compressed between the rigid outer cap and the adaptor;
the elastomeric inner cap is disposed on the tip of the container to sealingly close the fluid passageway; and/or
the elastomeric inner cap is axially compressed between protrusions of the rigid outer cap that extend radially inwardly and the tip of the container to sealingly close the fluid passageway.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1 is a cross-sectional view of an outer cap of a tip cap assembly;
FIG. 2 is a side view of the outer cap of FIG. 1;
FIG. 3 is a side view of an inner cap of the tip cap assembly;
FIG. 4 is a cross-sectional view of the outer cap of FIG. 1 including the inner cap of FIG. 3 disposed therein;
FIG. 5 is a cross-sectional view of a container having an adaptor disposed thereon; and
FIGS. 6 and 7 are a side view and a cross-sectional view, respectively, of a container having a tip cap assembly disposed thereon.

### DETAILED DESCRIPTION

As used herein, the term "proximal" refers to a location, such as a proximal end, that is nearer to a point of reference such as a point of contact of a user applying a force to a plunger rod of an injection device as described herein. As used herein, the term "distal" refers to a location, such as a distal end, that is farther from a point of reference such as a point of contact of the user applying a force to the plunger of the injection device as described herein. Thus, the terms "proximal" and "distal" refer to, for example, directions nearer to and farther from, respectively a user administering a medicinal fluid to a patient.

As used herein, the terms "axial," "axially," "longitudinal," and "longitudinally" generally mean and refer to a direction along or parallel to a longitudinal axis of an element(s) of the injection device described herein.

As used herein, the terms "radial," "radially," "lateral," and "laterally" generally mean and refer to a direction perpendicular to the central, longitudinal axis of the element(s) of the injection device described herein.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

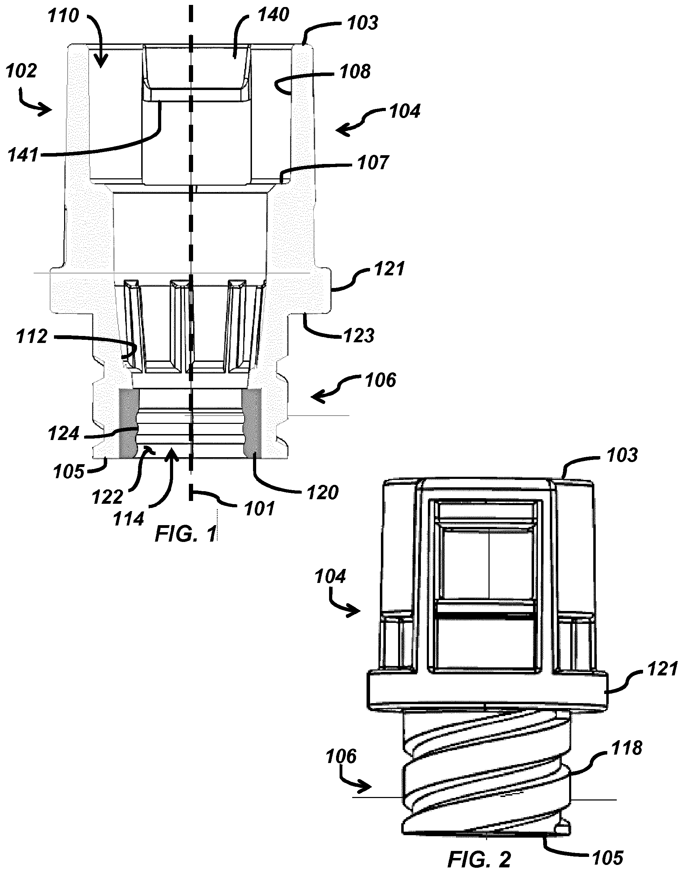

The tip cap assembly 100 disclosed herein comprises a rigid outer cap 102 and an inner cap 130. FIGS. 1 and 2 illustrates the rigid outer cap 102 of the tip cap assembly 100.

The outer cap 102 comprises a distal portion 104 and a proximal portion 106. The outer cap 102 has a longitudinal axis 101 extending between a distal end 103 and a proximal end 105 thereof. The outer cap 102 is a hollow body having an opening at each of the distal end 103 and the proximal end 105 thereof.

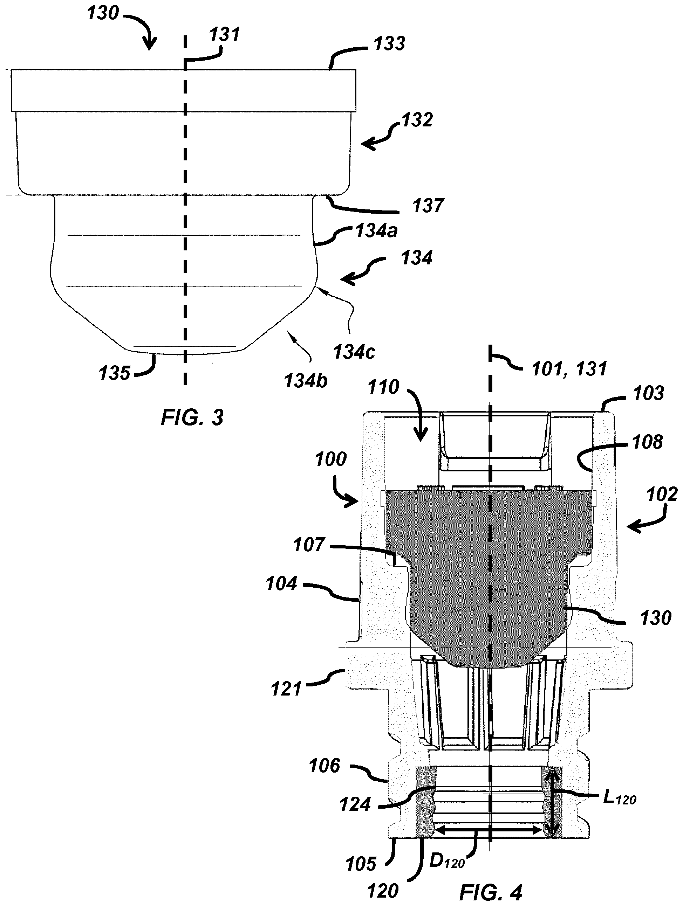

The distal portion 104 comprises a cavity 110 defined by an inner surface 108. As illustrated in FIG. 4, the cavity 110 is configured to retain an inner cap 130 therein. The inner surface 108 of the distal portion 104 decreases in width as the cavity 110 extends from the distal end 103 toward the proximal end 105. A shoulder 107 is defined by the inner surface 108 of the distal portion 104 at which the width of the cavity 110 is reduced in a stepped manner. The shoulder 107 is configured such that a portion of the inner cap 130 is disposed thereon when the inner cap 130 and the outer cap 102 are assembled together.

The distal portion 104 of the outer cap 102 may comprise at least one protrusion 140 proximate to the distal end 105. The protrusion(s) 140 extends radially inward toward and longitudinally along the longitudinal axis 101 of the outer cap 102. A proximal end 141 of the protrusion 140 may be referred to as an abutment surface and is configured to abut against a distal surface 133 of the inner cap 130 when the inner cap 130 is disposed therein.

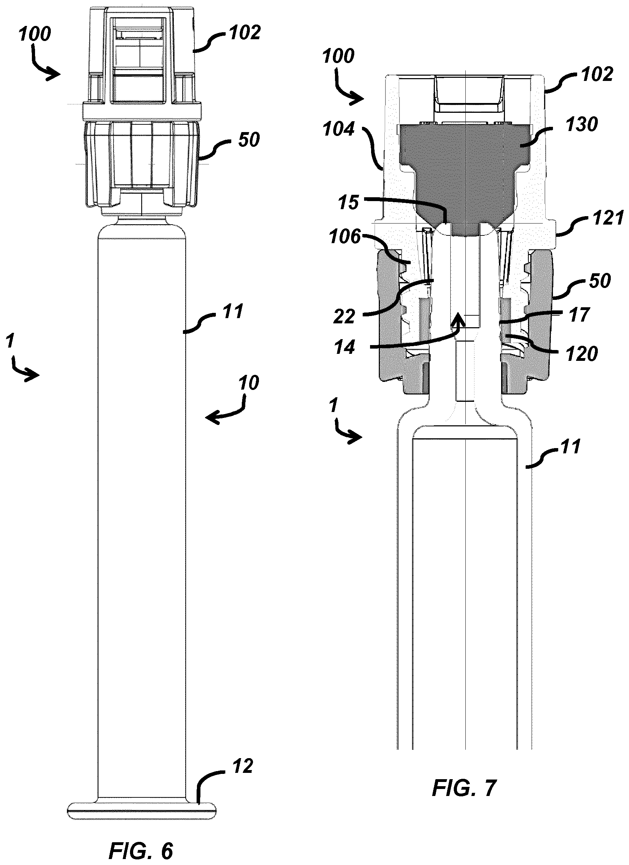

The proximal portion 106 comprises a cavity 114 defined by an inner surface 112 thereof. The cavity 114 of the proximal portion 106 is continuous with the cavity 110 of the distal portion 104. As illustrated in FIG. 7, the cavity 114 is configured to receive and surround a tip 22 of a container 10 therein when the tip cap assembly 100 is assembled with the container 10.

A sealing ring 120 is bonded to the inner surface 112 of the proximal portion 106 of the outer cap 102. The sealing ring 120 is made of a material such that the sealing ring 120 is radially compressible.

The sealing ring 120 is bonded to the inner surface 112 of the proximal portion 106 of the outer cap 102. The sealing ring 120 and proximal portion 106 may be bonded by a co-molding or overmolding process depending on the material compositions of the sealing ring 120 and outer cap 102. In a co-molding process, the sealing ring 120 and the outer cap 102 may be substantially simultaneously formed by an injection molding such that, as the molten materials of the sealing ring 120 and the outer cap 102 solidify after injection into a mold, the sealing ring 120 and the outer cap 102 are bonded together. In an overmolding process, the outer cap 102 may be injection molded and solidified prior to injection of the sealing ring 120 material into the mold, and the sealing ring 120 is bonded onto the outer cap 102 when the molten material of the sealing ring 120 solidifies.

The sealing ring 120 is made of an elastomeric material. The elastomeric material may be a thermoplastic elastomer. By way of non-limiting example, the sealing ring 120 may be made of natural rubber, synthetic rubber, thermoplastic elastomers, or combinations thereof.

The inner surface 122 of the sealing ring 120 is configured to directly engage the tip 22 of the container 10 when the tip cap assembly 100 is assembled with the container 20. The sealing ring 120 comprises a plurality of ribs 124 separately longitudinally by valleys, which are axially recessed surfaces relative to the ribs 124. As explained in further detail below, the surface of the ribs 124 (e.g., the surface area of the sealing ring 120 in contact with the tip 22) may be selected to modify the force to be applied by a machine and/or a user to axially displace the tip cap assembly 100 onto and/or off of the container 10.

Radially innermost surfaces of the ribs 124 engage a lateral surface of the tip 22 when the tip cap assembly 100 is assembled therewith. The radially innermost surfaces of the inner sealing ring 120 define an inner diameter D₁₂₀ of the sealing ring 120. The inner diameter D₁₂₀ of the sealing ring 120 is less than the outer diameter D₂₂ of the tip 22 of the container 20. The inner diameter D₁₂₀ of the sealing ring 120 is adjustable as the sealing ring 120 is radially compressible.

The proximal portion 106 comprises an outer surface 118 having threading thereon. The threaded outer surface 118 is configured to engage with threading of an adaptor 50 as discussed in further detail below.

The outer cap 102 is made of a rigid polymer. By way of non-limiting example, the outer cap 102 is made of polypropylene, polyethylene, polyvinylchloride, polystyrene, polycarbonate, acrylonitrile butadiene styrene or styrene acrylonitrile.

The outer cap 102 may also comprise a ring 121 defining a portion of the exterior surface 105 thereof. The ring 121 defines a proximal surface 123 that abuts against an adapter 50 when the tip cap assembly 100 is coupled thereto. The ring 121 may define the largest radial dimension of the outer cap 102.

FIG. 3 illustrates an inner cap 130 of the tip cap assembly 100, and FIG. 4 illustrates the inner cap 130 disposed within the outer cap 102.

The inner cap 130 comprises a distal portion 132 and a proximal portion 134. A longitudinal axis 131 of the inner cap 130 extends between a distal surface 133 and a proximal surface 135 of the inner cap 130.

When the inner cap 130 is disposed within the outer cap 102, the longitudinal axis 131 of the inner cap 130 may be coaxial with the longitudinal axis 101 of the outer cap 102.

The distal portion 132 may be generally cylindrical in shape with a planar distal surface 133. The proximal portion 134 may be have a first portion 134a that is generally cylindrical in shape immediately adjacent to the distal portion 132 and a second portion 134b that is frustoconical in shape at a proximal end thereof.

In some embodiments, the first portion 134a may have a bulbous portion that increases in width proximate to the second portion 134b. The bulbous portion is radially compressed when the inner cap 130 is disposed within the outer cap 102 in order to retain the inner cap 130 within the cavity 110. The proximal portion 134 comprises a planar proximal surface 135.

The distal portion 132 has a width (e.g., diameter) that is greater than a width of the proximal portion 134. The width of the proximal portion 134 and the distal portion 132 may such that the inner cap 130 is radially compressed by the inner surface 108 of the cavity 110 when the inner cap 130 is disposed in the outer cap 102.

The inner cap 130 is separately formed from and assembled with the outer cap 102. The inner cap 130 may be inserted into the outer cap 102 by passing the inner cap 130 through the opening at the distal end 103 of the outer cap 102 and into the cavity 110 of the distal portion 106 of the outer cap 102.

When the inner cap 130 is disposed within the outer cap 102, the distal surface 133 of the distal portion 132 may abut against the abutment surface 141 of the protrusion 140 of the outer cap 102. The proximal surface 137 of the distal portion 132 may abut against the shoulder 107 of the outer cap 102.

The inner cap 130 may be formed of an elastomeric material. Suitable materials for the inner cap 130 may include natural rubber, acrylate-butadiene rubber, cis-polybutadiene, chloro or bromobutyl rubber, chlorinated polyethylene elastomers, polyalkylene oxide polymers, ethylene vinyl acetate, fluorosilicone rubbers, hexafluoropropylene-vinylidene fluoride-tetrafluoroethylene terpolymers, butyl rubbers, polyisobutene, synthetic polyisoprene rubber, silicone rubbers, styrene-butadiene rubbers, tetrafluoroethylene propylene copolymers, thermoplastic-copolyesters, thermo-plastic elastomers, or the like or a combination thereof.

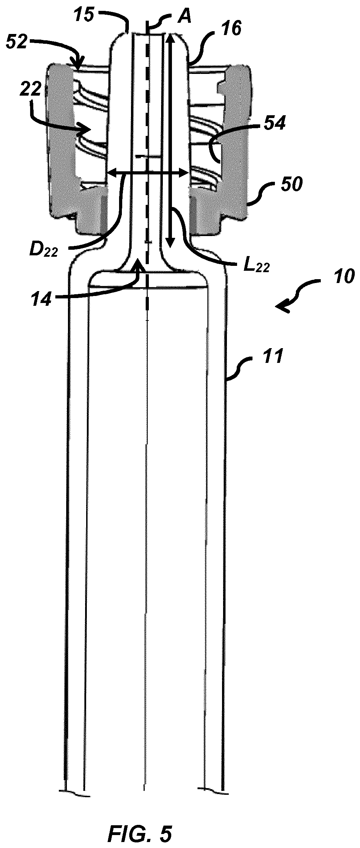

The outer cap 102 having the inner cap 130 disposed therein may be assembled with an injection device 1. FIGS. 5-7 show an injection device 1 in the form of a luer syringe according to an aspect of the disclosure. The tip cap assembly 100 of the present disclosure could be used with any other types of injection systems, such as a pen, or an infusion system, provided they include a distally projecting tip. For sake of clarity, the present disclosure will only be described with a luer syringe.

The injection device 1 includes a container 10. The container 10 comprises a barrel 11 extending axially along a longitudinal axis A. The container 10 is configured to contain a medicinal fluid therein. The barrel 11 comprises a flange 12 at a proximal end and a tip 22 at a distal end. The tip 22 includes a fluid passageway 14 extending therethrough, a distal surface 15 and a lateral surface 16 which is substantially tubular. While not illustrated in FIGS. 6 and 7, the injection device 1 may further comprise a stopper coupled to a plunger. In operation, a user depresses the plunger so to displace the stopper and dispense a medicinal fluid from the container 10 through the fluid passageway 14 of the tip 22.

The tip 22 may be configured to be connected to an additional element such as a needle hub or intravenous (IV) line, thereby allowing the medicinal fluid contained in the barrel 11 to be expelled and further injected into an intended target through the fluid passageway 14.

The barrel 11 and tip 22 may be made of glass or plastic.

An adaptor 50 is securely engaged with the tip 22. The adaptor 50 may be separately formed and fixedly attached to the injection device 1 by clipping, screwing, welding, an adhesive, or the like. Alternatively, the adaptor 50 may be integrally formed with the barrel 11 such as by molded.

The adaptor 50, which is best illustrated in FIG. 5, surrounds the tip 22 so as to define an annual space 52 therebetween for receiving the proximal portion 106 of the outer cap 102. An inner surface 54 of the adaptor 50 comprises threading to engage with threading on the outer surface 118 of the proximal portion 106 of the outer cap 102.

The adaptor 50 may be made of a plastic material. By way of non-limiting example, the adaptor 50 may be made of acrylonitrile butadiene styrene (ABS), polycarbonate (PC), polyoxymethylene (POM), polystyrene (PS), polybutylene terephthalate (PBT), polypropylene (PP), polyethylene (PE), polyamide (PA), thermoplastic elastomer (TPE) and their combinations.

The tip cap assembly 100 is coupled to the adaptor 50 by engaging the threaded outer surface 118 of the outer cap 102 with the threaded inner surface 54 of the adaptor 50. When assembled together, the proximal portion 106 of the outer cap 102 is disposed in the annular space 52 and surrounds the tip 22 of the injection device 1.

When assembled, the proximal surface 107 of the ring 121 of the outer cap 102 abuts against a distal end of the adapter 50.

When assembled, the inner cap 130 is axially compressed between the tip 22 of the injection device 1 and the abutment surface 141 of the protrusion(s) 140 of the outer cap 102. More particularly, the proximal surface 135 of the inner cap 130 abuts against the distal surface 15 of the tip 22 and the distal surface 133 of the inner cap 130 abuts against the abutment surface 141 of the protrusion 140 of the outer cap 102. The seating of the inner cap 130 between the protrusion 140 and the tip 22 maintains the inner cap 130 in position within the outer cap 102 during a sterilization process and during subsequent storage of the device 1 until a user is ready to use the device 1 to dispense medical fluid therefrom.

The proximal portion 134 of the inner cap 130 may partially protrude into the fluid passageway 14 of the injection device 1. The positioning of the inner cap 130 against the tip 22 seals the fluid passageway 14 of the injection device 1 and prevents fluid from flowing out of the injection device 1. The engagement of the inner cap 130 and the tip 22 also prevents any contamination of the medical fluid disposed within the container 10 from the outside environment, thereby assuring the container closure integrity.

In addition, the inner cap 130 is dimensioned to be radially compressed along at least a portion of its length. For example, the bulbous portion of the proximal portion 134 may be radially compressed to a greater degree relative to the remainder of the first portion 134a. Radially compression of the inner cap 130 maintains the inner cap 130 in position within the outer cap 102 within the outer cap 102 during a sterilization process and during subsequent storage of the device 1 until a user is ready to use the device 1 to dispense medical fluid therefrom. Maintaining the inner cap 130 within its seated position with the outer cap 102 maintains the fluid seal of the inner cap 130 and the tip 22 to avoid contamination of the medicinal fluid in the container 10.

The inner cap 130 is shaped and dimensioned such that the inner cap 130 does not surround the lateral surface 16 of the tip 22. Rather, the lateral surface 16 of the tip 22 is engaged with ribs 124 of the sealing ring 120 of the tip cap assembly 100. As the inner cap 130 does not engage with the lateral surface 16 of the tip 22 when the tip cap assembly 100 is assembled with the container, the inner cap 130 is less prone to displacement from its seated position within the cavity 112 of the rigid cap 102 compared to an inner cap having a proximal portion that is configured to at least partially surround the lateral surface 16 of the tip 22 when assembled therewith.

A seal may be formed between the radially innermost surfaces 122 of the ribs 124 of the sealing ring 120 and the lateral surface 16 of the tip 22 to ensure container closure integrity such that any contamination of the medical fluid disposed within the container 10 from the outside environment is inhibited.

Radially innermost surfaces 122of the ribs 124 directly contact the lateral surface 16 of the tip 22 of the container 10. When assembled, the sealing ring 120 of the tip cap assembly 100 is radially compressed between the rigid outer cap 102 and the adaptor 50 when the tip cap assembly 100 and injection device 1 are assembled together. The sealing ring 120 is radially compressed by virtue of the relative dimensions of the inner diameter D₁₂₀ of the sealing ring 120 and the outer diameter D₂₂ of the tip 22 on which the tip cap assembly 100 is disposed. More particularly, the inner diameter D₁₂₀ of the sealing ring 120 is less than the outer diameter D₂₂ of the tip 22. By way of non-limiting example, the inner diameter D₁₂₀ may be 5 to 20% less than the outer diameter D₂₂ of the tip 22.

The height L₁₂₀, or the axial dimension, of the sealing ring 120 is less than the height L₂₂ of the tip 22 of the container 10. In some embodiments, the height L₁₂₀ of the sealing ring 120 may be between 30 and 50% and, more particularly, 40% of the height L₂₂ of the tip 22 of the container 10. Valleys between the ribs 124 on the inner surface 122 of the sealing ring 120 are also provided to reduce the contact area between the sealing sing 120 and the tip 22, which also reduces the force necessary to remove the tip cap assembly 100 from the container 10. The surface area of the ribs 124, which defines the contact area of the sealing ring 120 in direct contact with the tip 22, may be selected to modify the force to be applied by a machine and/or a user to axially displace the tip cap assembly 100 onto and/or off of the container 10.

The provision of the sealing ring 120 made of a compressible material absorbs some of the compressive forces existing between the adaptor 50, the proximal portion 106 of the rigid cap 102, and the tip 22 when assembled together. By providing the compressible material of the sealing ring 120, the proximal portion 106, which is made of a rigid polymeric material, is less prone to cracking as a result of the compressive forces compared to a rigid cap having a proximal portion is a unitary piece of a rigid material having threading on an outer surface in contact with the adaptor 50 and ribs on an inner surface in contact with the lateral surface 16 of the tip 22. Cracking of the rigid cap 102 jeopardizes the container closure integrity. This tip cap assembly 100 according to the present disclosure is therefore less prone to cracking when the tip cap assembly 100 is assembled with the container 10 that is made of glass or plastic.

The tip cap assembly 100 is assembled onto the container 10 by engaging the threaded surface 188 of the outer cap 102 with the threaded inner surface 54 of the adaptor 50. As the tip cap assembly 100 is rotated to engage with the adaptor 50, the sealing ring 120 glides along lateral surface 16 of the tip 22 of the container 10. Various dimensions of the sealing ring 120 may be selected to control the frictional forces between the sealing ring 120 and the tip 22 and, thus, the torque force that is applied to engage and disengage the tip cap assembly 100 with the adaptor 50 and the container 10.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure. In addition, features from one embodiment may be combined with features of another embodiment while still being encompassed within the scope of the invention as contemplated by the inventors.

## Claims

1. A tip cap assembly (100) for closing a fluid passageway extending through a tip (22) of a container (20) of a medical injection device (10), the tip cap assembly (100) comprising:
a rigid outer cap (102) having a distal portion (104) and a proximal portion (106), wherein:
an inner surface (108) of the distal portion (104) defines a cavity (110) configured to retain an elastomeric inner cap (130) therein; and
an inner surface (112) of the proximal portion (106) defines a cavity (114) configured to surround the tip (22) of the container (20) when the tip cap assembly (100) is assembled with the container (20); and
the elastomeric inner cap (130) disposed within the cavity (110) of the distal portion (104) of
the rigid outer cap (102),
the tip cap assembly being **characterized in that** it further comprises:
an elastomeric sealing ring (120) bonded to the inner surface (112) of the proximal portion (106) of the rigid outer cap (102), an inner surface (122) of the sealing ring (120) comprising a plurality of ribs (124) having a surface (126) configured to engage a lateral surface (16) of the tip (22) when the tip cap assembly (100) is assembled with the container (20);
wherein a proximal surface (135) of the elastomeric inner cap (130) abuts against a distal surface of the tip (22) of the container (20) without engagement of the elastomeric inner cap (130) with the lateral surface (16) of the tip (22) when the tip cap assembly (100) is assembled with the container (20).

2. The tip cap assembly (100) of claim 1, wherein an inner diameter (D₁₂₀) of the sealing ring (120) is less than an outer diameter (D₂₂) of the tip (22) on which the tip cap assembly (100) is configured to be disposed.

3. The tip cap assembly (100) of any of claims 1-2, wherein a height (L₁₂₀) of the sealing ring (120) is less than a height (L₂₂) of the tip (22) on which the tip cap assembly (100) is configured to be disposed, preferably the height (L₁₂₀) of the sealing ring is 40% of the height (L₂₂) of the tip (22).

4. The tip cap assembly (100) of any of claims 1-3, wherein the elastomeric sealing ring (120) comprises a thermoplastic elastomer.

5. The tip cap assembly (100) of any of claims 1-4, wherein the rigid outer cap (102) is made of a rigid polymer, the rigid polymer preferably being polypropylene, polyethylene, polyvinylchloride, polystyrene, polycarbonate, acrylonitrile butadiene styrene or styrene acrylonitrile.

6. An injection device (1) comprising:
a container (20) having a barrel (24) and a tip (22) distally projecting from the barrel (24), the tip (22) comprising a fluid passageway (26) extending longitudinally therethrough;
an adaptor (50) disposed circumferentially about the tip (22) of the container (20); and
the tip cap assembly (100) of any of claims 1-5 coupled to the adaptor (50).

7. The injection device (1) of claim 6, wherein an outer surface (116) of the proximal portion (106) of the rigid outer cap (102) has a threaded surface (118).

8. The injection device (1) of claim 7, wherein the adaptor (50) comprises a distal portion (52) having an inner surface (54) that is threaded, the threaded surface (118) of the proximal portion (106) of the rigid outer cap (102) engaged with the threaded inner surface (54) of the adaptor (50).

9. The injection device (1) of any of claims 6-8, wherein the elastomeric sealing ring (120) of the tip cap assembly (100) is radially compressed between the rigid outer cap (102) and the adaptor (50).

10. The injection device (1) of any of claims 6-9, wherein the elastomeric inner cap (130) is disposed on the tip (22) of the container (20) to sealingly close the fluid passageway (26).

11. The injection device (1) of any of claims 6-10, wherein the elastomeric inner cap (130) is axially compressed between protrusions (140) of the rigid outer cap (102) that extend radially inwardly and the tip (22) of the container (20) to sealingly close the fluid passageway (26).

12. A method of forming the tip cap assembly of any of claims 1-5, comprising:
co-molding the sealing ring (120) to the inner surface (112) of the proximal portion (106) of the rigid outer cap (102), and
disposing the elastomeric inner cap (130) within the cavity (110) of the distal portion (104) of the rigid outer cap (102).

## Patentansprüche

1. Spitzenkappenanordnung (100) zum Schließen eines Fluiddurchlasses, der sich durch eine Spitze (22) eines Behälters (20) einer medizinischen Injektionsvorrichtung (10) erstreckt, wobei die Spitzenkappenanordnung (100) Folgendes umfasst:
eine starre Außenkappe (102) mit einem distalen Abschnitt (104) und einem proximalen Abschnitt (106), wobei:
eine Innenfläche (108) des distalen Abschnitts (104) einen Hohlraum (110) definiert, der dazu ausgelegt ist, eine elastomere Innenkappe (130) darin zu halten; und
eine Innenfläche (112) des proximalen Abschnitts (106) einen Hohlraum (114) definiert, der dazu ausgelegt ist, die Spitze (22) des Behälters (20) zu umgeben, wenn die Spitzenkappenanordnung (100) am Behälter (20) montiert ist; und
die elastomere Innenkappe (130), die im Hohlraum (110) des distalen Abschnitts (104) der starren Außenkappe (102) angeordnet ist,
wobei die Spitzenkappenanordnung **dadurch gekennzeichnet ist, dass** sie ferner Folgendes umfasst:
einen Elastomerdichtring (120), der an die Innenfläche (112) des proximalen Abschnitts (106) der starren Außenkappe (102) gebondet ist, wobei eine Innenfläche (122) des Dichtrings (120) eine Vielzahl von Rippen (124) umfasst, die eine Fläche (126) aufweisen, die dazu ausgelegt ist, in eine Seitenfläche (16) der Spitze (22) einzugreifen, wenn die Spitzenkappenanordnung (100) am Behälter (20) montiert ist;
wobei eine proximale Fläche (135) der elastomeren Innenkappe (130) an einer distalen Fläche der Spitze (22) des Behälters (20) ohne Eingriff der elastomeren Innenkappe (130) in die Seitenfläche (16) der Spitze (22) anliegt, wenn die Spitzenkappenanordnung (100) am Behälter (20) montiert ist.

2. Spitzenkappenanordnung (100) nach Anspruch 1, wobei ein Innendurchmesser (D₁₂₀) des Dichtrings (120) kleiner ist als ein Außendurchmesser (D₂₂) der Spitze (22), auf der die Spitzenkappenanordnung (100) ausgelegt ist, angeordnet zu sein.

3. Spitzenkappenanordnung (100) nach einem der Ansprüche 1 bis 2, wobei eine Höhe (L₁₂₀) des Dichtrings (120) kleiner ist als eine Höhe (L₂₂) der Spitze (22), auf der die Spitzenkappenanordnung (100) ausgelegt ist, angeordnet zu sein, wobei die Höhe (L₁₂₀) des Dichtrings vorzugsweise 40% der Höhe (L₂₂) der Spitze (22) beträgt.

4. Spitzenkappenanordnung (100) nach einem der Ansprüche 1 bis 3, wobei der Elastomerdichtring (120) ein thermoplastisches Elastomer umfasst.

5. Spitzenkappenanordnung (100) nach einem der Ansprüche 1 bis 4, wobei die starre Außenkappe (102) aus einem starren Polymer besteht, wobei das starre Polymer vorzugsweise Polypropylen, Polyethylen, Polyvinylchlorid, Polystyrol, Polycarbonat, Acrylnitrilbutadienstyrol oder Styrolacrilnitrit ist.

6. Injektionsvorrichtung (1), die Folgendes umfasst:
einen Behälter (20) mit einem Zylinder (24) und einer Spitze (22), die vom Zylinder (24) distal vorsteht, wobei die Spitze (22) einen Fluiddurchlass (26) umfasst, der sich längs dadurch erstreckt;
einen Adapter (50), der umfänglich um die Spitze (22) des Behälters (20) angeordnet ist; und
die Spitzenkappenanordnung (100) nach einem der Ansprüche 1 bis 5, die an den Adapter (50) gekoppelt ist.

7. Injektionsvorrichtung (1) nach Anspruch 6, wobei eine Außenfläche (116) des proximalen Abschnitts (106) der starren Außenkappe (102) eine Gewindefläche (118) aufweist.

8. Injektionsvorrichtung (1) nach Anspruch 7, wobei der Adapter (50) einen distalen Abschnitt (52) mit einer Innenfläche (54) umfasst, die ein Gewinde aufweist, wobei die Gewindefläche (118) des proximalen Abschnitts (106) der starren Außenkappe (102) mit der Gewindeinnenfläche (54) des Adapters (50) im Eingriff ist.

9. Injektionsvorrichtung (1) nach einem der Ansprüche 6 bis 8, wobei der Elastomerdichtring (120) der Spitzenkappenanordnung (100) zwischen der starren Außenkappe (102) und dem Adapter (50) radial zusammengedrückt ist.

10. Injektionsvorrichtung (1) nach einem der Ansprüche 6 bis 9, wobei die elastomere Innenkappe (130) auf der Spitze (22) des Behälters (20) angeordnet ist, um den Fluiddurchlass (26) abdichtend zu schließen.

11. Injektionsvorrichtung (1) nach einem der Ansprüche 6 bis 10, wobei die elastomere Innenkappe (130) zwischen Vorsprüngen (140) der starren Außenkappe (102), die sich radial nach innen erstrecken, und der Spitze (22) des Behälters (20) axial zusammengedrückt ist, um den Fluiddurchlass (26) abdichtend zu schließen.

12. Verfahren zum Bilden der Spitzenkappenanordnung nach einem der Ansprüche 1 bis 5, das Folgendes umfasst:
Anfügen des Dichtrings (120) mittel Co-Molding an die Innenfläche (112) des proximalen Abschnitts (106) der starren Außenkappe (102), und
Anordnen der elastomeren Innenkappe (130) im Hohlraum (110) des distalen Abschnitts (104) der starren Außenkappe (102).

## Revendications

1. Ensemble de capuchon d'embout (100) destiné à fermer un passage de fluide s'étendant à travers un embout (22) d'un récipient (20) d'un dispositif d'injection médical (10), l'ensemble de capuchon d'embout (100) comprenant :
un capuchon extérieur rigide (102) comportant une partie distale (104) et une partie proximale (106), dans lequel :
une surface interne (108) de la partie distale (104) définit une cavité (110) configurée pour retenir un capuchon interne élastomère (130) à l'intérieur ; et
une surface interne (112) de la partie proximale (106) définit une cavité (114) configurée pour entourer l'embout (22) du récipient (20) lorsque l'ensemble capuchon d'embout (100) est assemblé avec le récipient (20) ; et
le capuchon intérieur en élastomère (130) est disposé à l'intérieur de la cavité (110) de la partie distale (104) du capuchon extérieur rigide (102),
l'ensemble capuchon d'embout étant **caractérisé en ce qu'**il comprend en outre :
une bague d'étanchéité élastomère (120) liée à la surface interne (112) de la partie proximale (106) du capuchon externe rigide (102), une surface interne (122) de la bague d'étanchéité (120) comprenant une pluralité de nervures (124) ayant une surface (126) configurée pour s'engager avec une surface latérale (16) de l'embout (22) lorsque l'ensemble capuchon d'embout (100) est assemblé avec le récipient (20) ;
dans lequel une surface proximale (135) du capuchon intérieur en élastomère (130) vient en butée contre une surface distale de l'embout (22) du récipient (20) sans engagement du capuchon intérieur en élastomère (130) avec la surface latérale (16) de l'embout (22) lorsque l'ensemble capuchon d'embout (100) est assemblé avec le récipient (20).

2. L'ensemble capuchon d'embout (100) selon la revendication 1, dans lequel un diamètre intérieur (D₁₂₀ ) de la bague d'étanchéité (120) est inférieur à un diamètre extérieur (D₂₂ ) de l'embout (22) sur lequel l'ensemble capuchon d'embout (100) est configuré pour être disposé.

3. L'ensemble capuchon d'embout (100) de l'une quelconque des revendications 1 à 2, dans lequel la hauteur (L₁₂₀ ) de la bague d'étanchéité (120) est inférieure à la hauteur (L₂₂ ) de l'embout (22) sur lequel l'ensemble capuchon d'embout (100) est configuré pour être disposé, de préférence la hauteur (L₁₂₀ ) de la bague d'étanchéité est égale à 40 % de la hauteur (L₂₂ ) de l'embout (22).

4. Ensemble capuchon d'embout (100) selon l'une quelconque des revendications 1 à 3, dans lequel la bague d'étanchéité élastomère (120) comprend un élastomère thermoplastique.

5. Ensemble capuchon d'embout (100) selon l'une quelconque des revendications 1 à 4, dans lequel le capuchon extérieur rigide (102) est constitué d'un polymère rigide, le polymère rigide étant de préférence du polypropylène, du polyéthylène, du chlorure de polyvinyle, du polystyrène, du polycarbonate, de l'acrylonitrile butadiène styrène ou du styrène acrylonitrile.

6. Dispositif d'injection (1) comprenant :
un récipient (20) comportant un cylindre (24) et une pointe (22) faisant saillie distalement à partir du cylindre (24), la pointe (22) comprenant un passage de fluide (26) s'étendant longitudinalement à travers celle-ci ;
un adaptateur (50) disposé circonférentiellement autour de l'embout (22) du récipient (20) ; et
l'ensemble capuchon d'embout (100) de l'une quelconque des revendications 1 à 5 couplé à l'adaptateur (50).

7. Dispositif d'injection (1) selon la revendication 6, dans lequel une surface extérieure (116) de la partie proximale (106) du capuchon extérieur rigide (102) comporte une surface filetée (118).

8. Dispositif d'injection (1) selon la revendication 7, dans lequel l'adaptateur (50) comprend une partie distale (52) présentant une surface intérieure (54) qui est filetée, la surface filetée (118) de la partie proximale (106) du capuchon extérieur rigide (102) s'engageant avec la surface intérieure filetée (54) de l'adaptateur (50).

9. Dispositif d'injection (1) selon l'une quelconque des revendications 6 à 8, dans lequel la bague d'étanchéité élastomère (120) de l'ensemble capuchon d'embout (100) est comprimée radialement entre le capuchon extérieur rigide (102) et l'adaptateur (50).

10. Dispositif d'injection (1) selon l'une quelconque des revendications 6 à 9, dans lequel le capuchon intérieur en élastomère (130) est disposé sur l'embout (22) du récipient (20) afin de fermer de manière étanche le passage de fluide (26).

11. Dispositif d'injection (1) selon l'une quelconque des revendications 6 à 10, dans lequel le capuchon intérieur élastomère (130) est comprimé axialement entre des saillies (140) du capuchon extérieur rigide (102) qui s'étendent radialement vers l'intérieur et l'embout (22) du récipient (20) afin de fermer de manière étanche le passage de fluide (26).

12. Procédé de formation de l'ensemble embout-capuchon selon l'une quelconque des
revendications 1 à 5, comprenant :
le co-moulage de la bague d'étanchéité (120) sur la surface interne (112) de la partie proximale (106) du capuchon externe rigide (102), et
la mise en place du capuchon intérieur en élastomère (130) dans la cavité (110) de la partie distale (104) du capuchon extérieur rigide (102).
